(19) Europäisches Patentamt
European Patent Office
Office européen des brevets

(11) Numéro de publication: 0 107 577
B1

(12) # FASCICULE DE BREVET EUROPÉEN

(45) Date de publication du fascicule du brevet: **14.01.87**

(51) Int. Cl.⁴: **C 07 C 119/048**

(21) Numéro de dépôt: **83401997.8**

(22) Date de dépôt: **13.10.83**

(54) **Isocyanates à structure diphénylméthane et leur procédé de fabrication.**

(30) Priorité: **19.10.82 FR 8217443**

(43) Date de publication de la demande: **02.05.84 Bulletin 84/18**

(45) Mention de la délivrance du brevet: **14.01.87 Bulletin 87/3**

(84) Etats contractants désignés: **AT BE CH DE FR GB IT LI LU NL SE**

(56) Documents cité:
**EP-A-0 024 665**
**EP-A-0 046 556**
**FR-A-1 447 964**
**US-A-3 180 883**

(73) Titulaire: **ATOCHEM, 4 & 8, Cours Michelet La Défense 10, F-92800 Puteaux (FR)**

(72) Inventeur: **Kervennal, Jacques, 134, Rue E. Locard, F-69005 Lyon (FR)**
Inventeur: **Commandeur, Raymond, "Le Rocher" Avenue de Venaria, F-38220 Vizille (FR)**

(74) Mandataire: **Foiret, Claude, ATOCHEM Département Propriété Industrielle, F-92091 Paris la Défense 10 Cédex 42 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 107 577 B1

LIBER, STOCKHOLM 1987

**Description**

L'invention concerne de nouveaux isocyanates aromatiques obtenus sous forme de mélanges d'isomére et correspondant à des structures hydrocarbonées dissymétriques diphénylméthanes substituées, éventuellement en association avec des structures dissymétriques diphényléthanes substituées, le ou les substituants étant un ou plusieurs groupements alkyles méthyle ou éthyle. Les structures hydrocarbonées sont obtenues à partir des isomères de xylène. Ces isocyanates sont utilisables dans la fabrication de polyuréthannes.

Des isocyanates de structure aromatique hydrocarbonée diphénylméthane sont déjà synthétisés industriellement il s'agit du diphénylméthane diisocyanate (MDI), commercialisé, soit sous forme d'un mélange de diisocyanates et de polyisocyanates, soit sous forme pure après distiliation. Le constituant principal du MDI est le diisocyanate-4,4' diphénylméthane qui est solide à température ordinaire; ainsi, suivant sa teneur, il peut être nécessaire de liquéfier le mélange d'isocyanates, avant utilisation, soit en le fondant, soit en le traitant chimiquement. Les isocyanates de l'invention, qui se présentent sous forme de mélanges d'isomères, liquides à température ordinaire ou à bas point de fusion, permettent d'éviter ces traitements préalables.

Les structures isocyanates conformes à l'invention se présentent sous forme des formules générales suivantes:

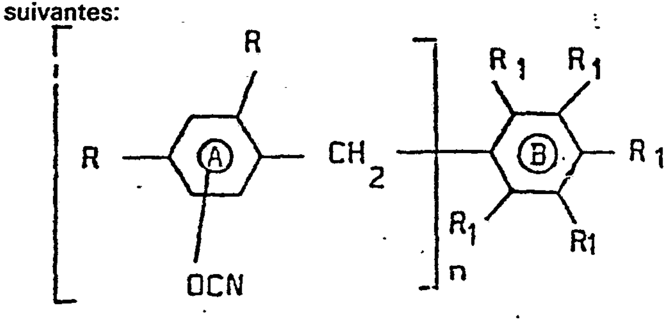

et

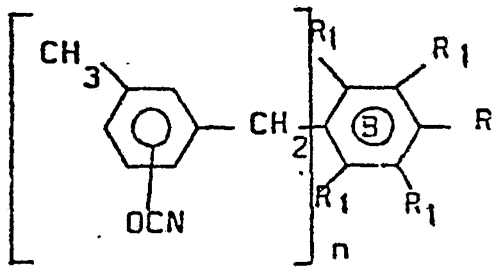

I II

seules ou en mélange, éventuellement en association avec

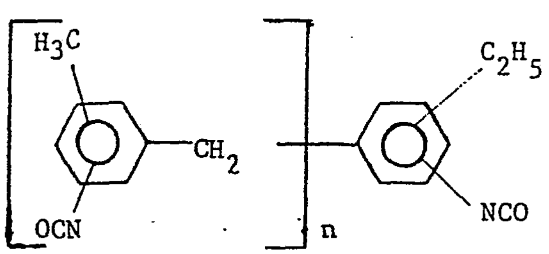

+

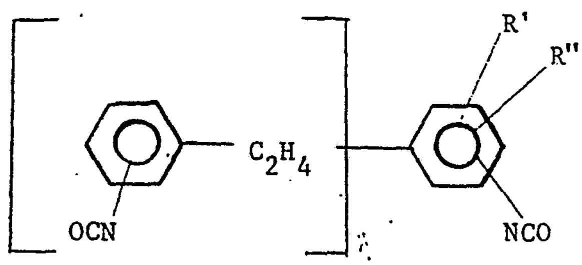

III IV

Avec n de 1 à 2; R = H ou $CH_3$ et $R_1$ = H, $CH_3$ ou NCO.

Dans les noyaux de type A, un seul R correspond à CH et dans les noyaux de type B, un seul R, correspond à NCO et deux $R_1$ correspondent à $CH_3$ sachant que sui cenoyau est fixée une lunction - NCO ne pouvant être entourée par desgroupes méthyle sur deux positions en outho.

R' = $CH_3$ ou $C_2H_5$

R" = H ou $CH_3$ avec R" = H si R' = $C_2H_5$ et R" = $CH_3$ si R' = $CH_3$.

Des diisocyanates de structures voisines ont été décrits dans le US.A. 3.180.883, mais ils concernent des produits à fonction -NCO située entre deux groupements aryles en position ortho par rapport à ladite fonction -NCO permettant d'abaisser la réactivité de cette fonction. Une telle position de la fonction -NCO n'est pas envisageable dans le cas de la présente demande dont le but est d'obtenir un produit de réactivité équivalente à celle des produits connus tout en abaissant par rapport à eux sa tension de vapeur par substitution sur la molécule de diphénylméthane dilsocyanate trois groupements $-CH_3$. Cet abaissement de tension de vapeur permet d'obtenir des diisocyanates moins volatils et par conséquent moins toxiques. Dans le EP.A 0.024.665 sont décrits des diphénylméthanes diisocyanates monométhylés, mais ils restent toujours associés à au moins 30 % de diphénylméthane diisocyanate pur. Les mélanges selon cet EP.A 0024 665 restent toujours plus volatils que ceux de la présente demande et conservent les inconvénients de toxicité cités, ne serait-ce que par la présence du diphénylméthane diisocyanate pur.

La chloration photochimique ménagée d'un isomère de xylène conduit à un chlorure de benzyle substitué qu'on peut condenser par réaction de type FRIEDEL et CRAFTS sur un excès du xylène; il se forme suivant les

cas un isomère ou un mélange d'isomères diphénylméthanes substitués qu'on peut séparer du mélange réactionnel par distillation. Ainsi, la condensation de chlorure d'orthométhyl benzyle sur l'orthoxylène forme les deux isomères:

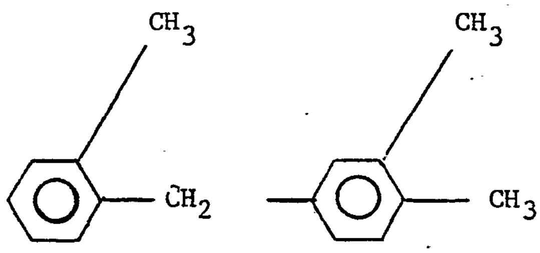 et 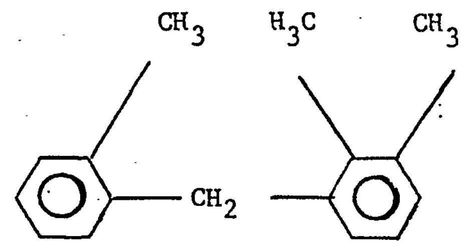

V VI

De même, la condensation de chlorure de méta méthyl benzyle sur du métaxyléne conduit aux deux isomères:

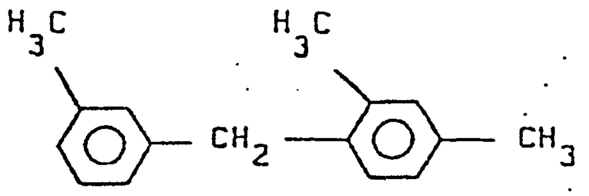 et 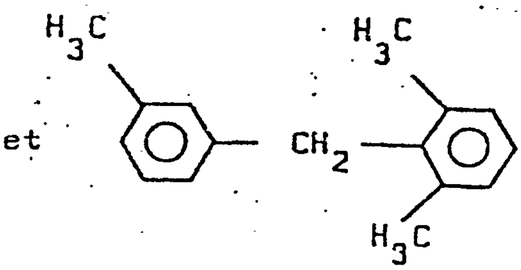

VII VIII

La condensation de chlorure de paraméthylbenzyle sur du paraxylène aboutit a un seul isomère

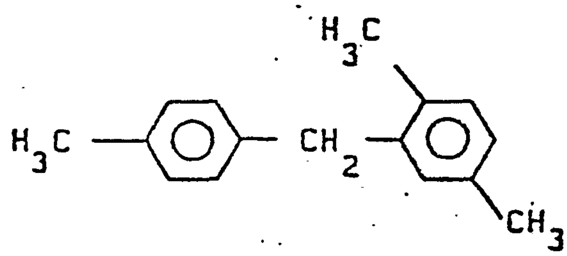

IX

Il est aussi possible de condenser un dérivé d'un isomère de xylène sur un autre isomère: ainsi, le chlorure d'orthométhylbenzyle réagit sur le parexyléne pour former un isomère:

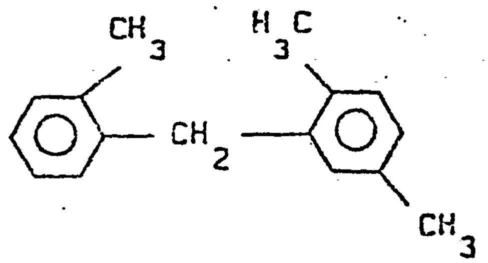

X

Ce type de réaction peut être étendu à des mélanges des isomères ortho, para, méta des xylènes ou aux mélanges commerciaux des xylènes. Dans ce dernier cas, la présence d'en viron 20 % d'éthylbenzène conduit à la formation de structures diphényléthanes. Suivant les conditions opératoires, en réglant l'excès de xylène, on obtient essentiellement des produits de condensation à deux noyaux aromatiques, mais il se forme aussi des produits à trois noyaux, l'ensemble constituant un mélange représenté par la ou les formules générales suivantes:

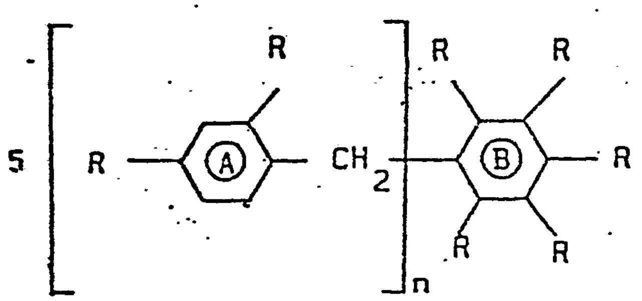

et

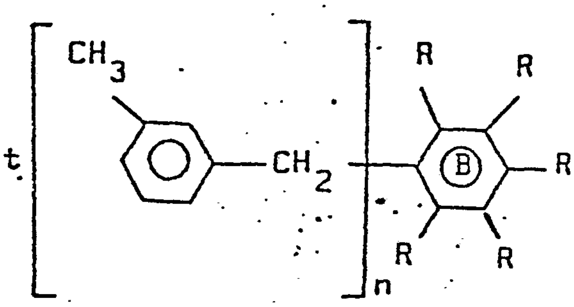

XI XII

seules ou en mélange, éventuellement en association avec

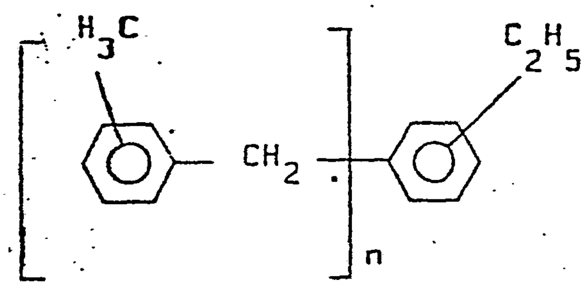

+

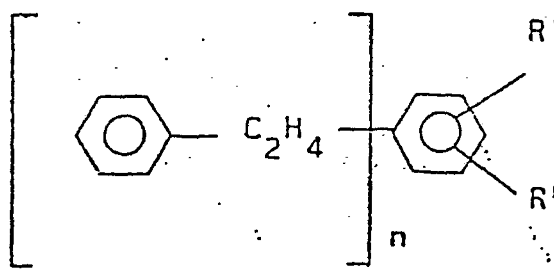

XIII XIV

Avec n de 1 à 2
R = H ou $CH_3$
Dans les noyaux de type A, un seul R correspond à $CH_3$ et dans les noyaux de type B, deux R correspondent à $CH_3$.
R' = $CH_3$ ou $C_2H_5$
R" = H ou $CH_3$ avec R" = H si R' = $C_2H_5$ et
R" = $CH_3$ si R' = $CH_3$
Les 2 dernières formules sont liées à la présence possible d'éthylbenzène dans les isomères de xylène.

Le mélange des isomères à deux noyaux peut être séparé par distillation du mélange réactionnel; cependant, il peut s'avérer économiquement avantageux d'effectuer la synthèse des isocyanates sur le mélange réactionnel brut. En pratique, celui-ci contient entre 70 et 95 % ds condensats diarylés. Pour effectuer ces réactions de condensation de FRIEDEL et CRAFTS avec de bons rendements, il est possible de se reporter aux descriptions faites dans le brevet français n° 2 432 199 et le certificat d'addition n° 2 449 954.

La synthèse des isocyanates correspondants fait appel à trois étapes réactionnelles successives à partir des hydrocarbures aromatiques: une de nitration, une d'hydrogénation et une de phosgénation.

On utilise pour la nitration un mélange d'acide nitrique et d'acide sulfurique concentrés à, au moins 85 % en poids et de préférence au moins 95 %. L'acide nitrique peut être placé en quantités stoechiométriques par rapport au composé aromatique et on utilise donc une mole d'acide per noyau aromatique à nitrer. Il est cependant avantageux d'opérer en presence d'un excès d'acide nitrique pouvant aller jusqu'à 20 % par rapport à la stoechiométrie. L'acide sulfurique peut être utilisé en quantité équimolaire par rapport à l'acide nitrique, mais on peut le placer en excès ou en défaut. La réaction de nitration peut s'effectuer entre 0°C et la température d'ébullition des mélanges, habituellement entre 0°C et 50°C, le composé aromatique étant de préférence solubilisé dans un solvant tel que le chlorure de méthylène. L'addition du mélange d'acides peut donc se faire à 0°C, mais il n'est pas gênant d'opérer à température ambiante à condition de contrôler l'exothermicité de la réaction. Celle-ci peut se poursuivre a température ambiante, mais il est préférable d'opérer au reflux du mélange, ce qui permet d'éliminer en partie l'excès d'acide nitrique. La phase organique, supérieure, est ensuite séparée des acides, neutralisée et évaporée à sec. Il est recommandé d'opérer sous atmosphère d'azote, dans un réacteur muni de moyens d'agitation efficace et de régulation de température.

La deuxième étape constitue l'hydrogénation des dérivés nitrés en amines correspondantes; elle peut être chimique, mais il est préférable d'opérer sous pression d'hydrogène, dans un réacteur résistant à la pression, muni d'une agitation et possédant les moyens de contrôle et régulation classiques, en présence d'un catalyseur à base de nickel, palladium, platine, ruthénium et autres.

Dans ce cas, on utilise un réacteur d'hydrogénation permettant de travailler sous des pressions pouvant atteindre 100 bars. La réaction peut s'effectuer sans solvant à une température où le dérivé nitré est fondu, ou dans des solvants classiques d'hydrogénation tels que les alcools, le dioxane, les éthers de l'éthylène glycol et autres.

On utilise préférentiellement un catalyseur constitué de palladium déposé sur support à des teneurs comprises entre 1 et 10 %, ce qui permet d'opérer à des températures comprises entre 30 et 100°C et des pressions de 20 à 50 bars. Le rapport molaire

$$\frac{\text{dérivé dinitré}}{\text{palladium}}$$

n'est pas impérativement fixé, mais il est de préférence compris entre 200 et 3 000. Après réaction et filtration du catalyseur, le solvant éventuellement utilisé est évaporé et le mélange des isomères de diamines obtenues peut être utilisé tel quel ou distillé. La troisième étape fait appel à des techniques classiques de phosgénation dans un réacteur muni d'une agitation et surmonté d'un réfrigérant. Par exemple, le mélange d'amines est placé dans un solvant aromatique chloré tel que le monochlorobenzène ou l'orthodichlorobenzène contenant la quantité nécessaire de phosgène, en maintenant la température aux environs de 0°C. On chauffe ensuite la suspension progressivement, le mélange réactionnel s'homogénéise entre 45 et 60°C. On continue d'élever lentement la température jusqu'au point d'ébullition du mélange, puis on distille le solvant de façon à récupérer les isocyanates formés. Ces derniers peuvent être distillés, s'ils sont suffisamment volatils, ou utilisés tels quels. Le mélange d'amines est de préférence placé dans le solvant à une concentration de 5 à 20 % et le phosgène est introduit en quantités stoechiométriques ou en léger excès. Une autre technique consiste à introduire conjointement dans le réacteur une solution portée au-dessus de 100°C dss amines dans un solvant aromatique chloré et un courant de phosgène solubilisé dans le même solvant qu'on amène à 120°C ou plus. La réaction est alors pratiquement immédiate.

Les isocyanates ainsi obtenus peuvent conduire, selon les techniques connues, à des polyuréthannes possédant de bonnes propriétés.

Les hydrocarbures de départ ainsi que les dérivés nitrés sont analysés par chromatographie en phase gazeuse (C.P.V.) et dans le cas de dérivés d'isomères purs de xylène, par résonance magnétique nucléaire (R.M.N. de $^{1}H$ et $^{13}C$). Nous avons utilisé un chromatographe PACKARD de type 427 à détection par ionisation de flamme muni d'une colonne OV 101 de 15 mètres de long en verre ordinaire et désactivée HMDS selon GROB. L'analyse se fait avec une température fixe de colonne de 140°C

Dans le cas des produits de nitration, nous avons utilisé la même colonne mais avec une programmation de température de 3°/mn entre 180 et 280°C.

Pour les amines, nous avons utilisé une colonne en verra de 2 mm de diamètre intérieur et de 2 mètres de longueur, emplie de support chromosorb W.N.A.M. 60-80 mesh. Johns MANVILLE, imprégnée à 5 % de KOH et 5 % d'Apiézon N-Société Apiézon products Limited - en opérant en isotherme à 220°C. Les amines ont également été analysées en chromatographie par perméation de gel sur colonne Shodex A 802 (éluant: tétrahydrofuranne).

**Exemple 1**

On chlore photochimiquement du para-xylène en l'irradiant à 80° C par une lampe actinique PHILLIPS TLADK de 30 watts et en limitant sa conversion à 10 %. On obtient un mélange, contenant l'excès de p-xylène et le chlorure de p-méthylbenzyle, qu'on fait réagir par condensation de FRIEDEL et CRAFTS en le coulant dans un pied de cuve de para-xylène à 100° C contenant du chlorure ferrique comme catalyseur. On prolonge la réaction jusqu'à la fin du dégagement d'HCl. On forme ainsi un mélange de p-xylène en excès et de produits de condensation à deux noyaux et à trois noyaux et plus. Après élimination du catalyseur on sépare par distillationle para méthyl benzyle - paraxylène, correspondant à la formule IX de la description, qui est ainsi obtenu pur et contrôlé par R.M.N. et C.P.V.

On dissout 42 g (0,2 mole) de l'hydrocaroure aromatique ainsi obtenu dans 80 ml de chlorure de méthylène. En maintenant la température entre 5 et 15° C, sous agitation, on coule en une demi-heure un mélange de 31 g d'acide nitrique à 98,7 % et 40 ml d'acide sulfurique à 96 %. Dès la fin de l'addition, on porte à reflux pendant 3 h 30, puis refroidit. Deux phases se séparent, l'une inférieure acide, l'autre supérieure organique. On extrait la phase organique et lave deux fois les acides avec du chlorure de méthylène. On réunuit les différentes phases organiqués, les neutralise avec du carbonate de potassium et évapore le solvant. On récupère 53 g de liquide visqueux jaune, contenant cinq isomères principaux de dinitration, l'isomère majoritaire ayant la structure suivante:

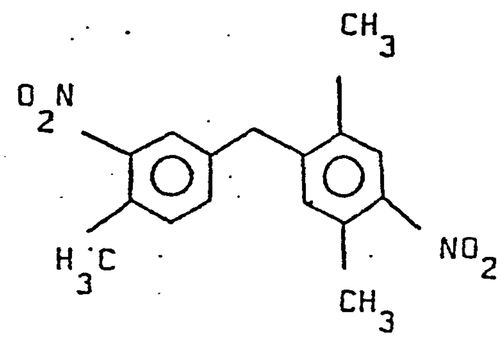

L'analyse par C.P.V. couplée avec la spectrométrie de masse met en évidence que ce produit ne contient que les isomères de dinitration avec de légères traces d'isomères de trinitration; ces derniers sont indécelables en R.M.N., les rapports de protons étant de 5 aromatiques pour 2 de nature méthylène et 9 de nature méthyle.

On introduit 21 g de ce mélange dans un autoclave en inox, muni d'une agitation magnétique, avec 0,8 g de catalyseur commercial constitué de palladium déposé sur charbon à 5 % et on complète le volume total à 0,2 l à l'aide de méthanol. Après balayage à l'azote, on introduit 35 bars d'hydrogène dans le réacteur et chauffe à 70° C sous agitation en maintenant la pression auto ur de la valeur initiale. Après 1 h 45 d'hydrogénation, la réaction étant finie, on décharge l'autoclave, filtre le catalyseur, évapore le méthanol et récupère 15,8 g d'un produit visqueux brun-foncé contenant le mélange des diamines attendues. On ajoute 14 g de ce produit d'hydrogénation à une solution de 35 g de phosgène dans 100 ml d'orthodichlorobenzène en maintenant la température autour de 10° C. On laisse réagir 1 heure à température ambiante, puis chauffe 1 heure à 45° C, 1 heure à 60° C et une demi-heure à 100° C. A ce moment, le mélange réactionnel s'homogénéise totalement et on porte à 140° C pendant 2 heures en faisant passer un courant d'azote afin de chasser le phosgène en excès et l'acide chlorhydrique formé. Puis on évapore le solvant sous vide et recueille 15,7 g d'un mélange d'isocyanates se présentant sous forme d'un liquide brun, teneur en NCO: 6,35 fonctions par kilo. La distillation de ce produit à 180° C sous 2 mm de Hg conduit à un solide de couleur orange-clair de point de fusion: 30 - 32° C, constitué des isomères de (paraméthylbenzyle isocyanate) - paraxylène isocyanate, teneur NCO = 2 fonctions par mole, correspondant à la formule générale 1 de la description avec n = 1.

**Exemple 2**

Dans 40 ml de chlorure de méthylène on dissout 21 g (0,1 mole) des 2 isomères d'(orthométhylbenzyl)-orthoxylène représentés par les formules V et VI de la description, obtenus dans les pourcentages respectifs de 77 et 23 et par condensationde FRIEDEL et CRAFTS, dans des conditions analogues à celles de l'exemple 1, du chlorure d'orthométhylbenzyle sur de l'orthoxylène. En opérant selon le mode opératoire décrit dans l'exemple 1, en utilisant un mélange nitrant constitué de 15,5 g d'acide nitrique à 98,7 % et 20 ml d'acide sulfurique à 96 %, on récupère 27 g de liquide visqueux brun contenant les isomères de dinitration de l'(orthométhylbenzyl)orthoxylène. L'analyse par résonance magnétique nucléaire, montre que le rapport des différents protons correspond à la théorie: on trouve ainsi 2 protons $CH_2$ pour 9 protons $CH_3$ et 5 protons

aromatiques. On hydrogène 21 g en présence de 0,8 g de catalyseur Pd sur charbon à 5 % dans 0,2 litre de méthanol sous 40 bars d'hydrogène à 60°C. Après filtration du catalyseur et distillation du méthanol, on recueille ainsi 16 g de produit brut contenant les isomères de la diamine attendue. La phosgénation, dans les conditions décrites dans l'exemple 1, de 15 g de ce produit brut donne, après distillation du solvant orthodichlorobenzène, 17,5 g d'un mélange liquide d'isocyanates, teneur en NCO: 6,58 fonctions par kg, qui, distillé sous vide à 180 - 185°C sous 2 mm Hg, fournit unmélange des isomères (orthométhylbenzyl isocyanate) orthoxyléne isocyanate, teneur en NCO: 2 fonctions par mole, se présentant sous forme d'un liquide jaune et correspondant à la formule générale 1 de la description dans laquelle n = 1.

**Exemple 3**

On dissout dans 80 ml de chlorure de méthylène 42 g (0,2 mole) du mélange des deux isomères de métaméthylbenzyle-métaxylène VII et VIII de la description obtenus dans les pourcentages respectifs de 78 et 22 par condensation de FRIEDEL et CRAFTS de chlorure de métaméthylbenzyle sur du métaxylène en excès, en opérant de la même façon que dans l'exemple 1. La nitration s'effectue comme dans l'exemple 1 en utilisant un mélange de 31 g d'acide nitrique à 98,7 % et de 40 ml d'$H_2SO_4$ à 96 %. On obtient, après extraction, neutralisation et distillation du solvant, 56,5 g de mélange de nitration se présentant sous forme d'un liquide de couleur jaune-clair. Le mélange ne contient pas de produit de mononitration; on peut déceler 9 isomères de dinitration et des traces disomères de trinitration, l'espéce principale, environ 35 % du total, répondant à la formule:

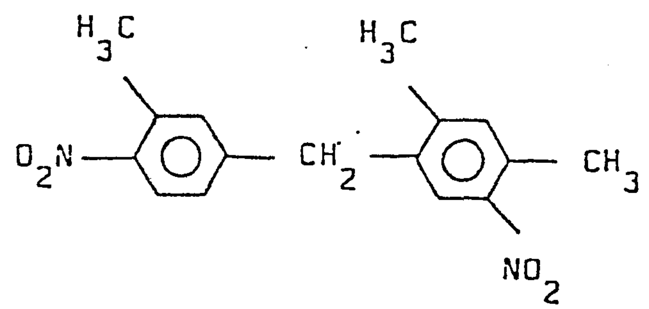

On hydrogène 22 g du produit dans 0,2 1 de méthanol, en présence de 0,8 g de palladium sur charbon à 5,% en opérant à 70°C sous 35 bars d'hydrogène pendant 1 h 30. On récupère, après refroidissement et filtration du catalyseur, 17 g de produit visqueux brun-foncé contenant les isomères de la diamine. La phosgénation de 15 g, selon la description faite dans l'exemple 1, donne, après distillation du solvant orthodichlorobenzène, 15,7 g de liquide correspondant au mélange des isomères (métaméthylbenzyl isocyanate)-métaxylène isocyanate attendus, teneur NCO: 2 équivalents par mole. La distillation de ce produit conduit à unliquide jaune-clair et correspond à la formule générale II de la description avec n = 1.

**Exemple 4**

Dans 80 ml de chlorure de méthylène, on dissout 42 g (0,2 mole) de l'isomère pur (d'orthométhyl benzyle) paraxylène correspondant à la formule X de la description préparé par condensation de FRIEDEL et CRAFTS de chlorure d'orthométhyl benzyle sur du paraxylène. En opérant de la même façon que dans l'exemple 1, on nitre l'hydrocarbure en utilisant un mélange de 31 g d'acide nitrique à 98,7 % et de 40 ml d'acide sulfurique à 96 %. On récupère après extraction, neutralisation et distillation du solvant, 59 g d'un mélange liquide orange, contenant 6 isomères principaux de dinitration. On hydrogène 20 g de ce produit dans 0,2 l de méthanol en présence de 0,8 g de palladium déposé à 5 % sur charbon, en opérant à 70°C sous 35 bars de pression d'hydrogène pendant 1 h 45. Après filtration et évaporation du solvant, on obtient 15,6 g d'un produit brun visqueux qui contient, d'après les résultats de spectrométrie de masse et de chromatographie par perméation de gel les isomères diaminés attendus. La phosgénation de 13,7 g de ce mélange, à l'aide de 34,2 g de phosgène, dans 100 ml d'orthodichlorobenzène, conduit, en opérant comme dans l'exemple 1 à 16,2 g de liquide renfermant les isomères d'(orthométhyljenzyl isocyanate)paraxylène isocyanate, teneur en NCO: 2 équivalents par mole. La distillation à 175°C sous 2 mm Hg, conduit à un liquide visqueux de couleur orango correspondant à la formule générale I de la description dans laquelle n = 1.

**Exemple 5**

La chloration photochimique ménagée de xylène commercial contenant 20 % d'éthylbenzène conduit, selon le mode opératoire de l'exemple 1, à un mélange contenant l'excès de xylène, les chlorures de méthylbenzyle, le chlorure de phénéthyle et le chloro-1 phényl-1 éthane. La condensation de FRIEDEL et CRAFTS sur du xylène commercial en excés forme des produits de condensation à deux noyaux et à trois noyaux et plus qu'on sépare par distillation. Les produits à deux noyaux renferment des isomères de (méthylbenzyle)-xylène et de diphényléthanes substitués répondant aux formules générales XI, XII, XIIL, XIV de la description dans lesquelles n = 1.

On dissout 21 g de ce mélange d'hydrocarbures à 2 noyaux aromatiques dans 40 ml de chlorure de méthylène en opérant comme dans l'exemple 1, on nitre à l'aide de 15,5 g d'acide nitrique à 98,7 % et 13,5 ml d'acide sulfurique à 96 %. Après extraction, neutralisation et distillation du solvant, on récupère 28 g de produit visqueux jaune-orange contenant les isomères de dinitration.

On hydrogène 20 g de ce mélange dans 0,2 l de méthanol en présence de 0,4 g de palladium déposé sur charbon à 5 % en opérant à 60°C sous 45 bars d'hydrogène pendant une heure. Après filtration du catalyseur et distillation du solvant, on recueille 45 g d'un produit visqueux brun-foncé renfermant le mélange des diamines attendues. On ajoute 14 g de ce produit d'hydrogénation à une solution de 37 g de phosgène dans 140 ml d'orthodichlorobenzène et on opère selon la description faite dans l'exemple 1. Après évaporation sous pression réduite du solvant, on récupère 16 g de mélange d'isocyanates, teneur en NC0: 6,5 fonctions par kilo. Une distillation portant sur 15 g de ce mélange permet d'obtenir 11 g d'un liquida jaune pâle correspondant aux formules générales I, II, III, IV de la description avec n = 1, teneur NCD: 2 fonctions par mole.

**Exemple 6**

On dissout dans 40 ml de chlorure de méthylène 31,4 g du mélange de produits essentiellement à trois noyaux aromatiques, résultant de la condensation de FRIEDEL et CRAFTS décrite dans l'exemple 5 et séparés des isomères à deux noyaux. On opère comme dans l'exemple 1 en utilisant 23,25 g d'acide nitrique à 98,7 % et 30 ml d'acide sulfurique à 96 % On obtient ainsi, après extraction, neutralisation et distillation du solvant, 42,5 g de mélange brut de nitration dont on hydrogène 20 g dans 0,2 litre de méthanol, en présence de 0,8 g de catalyseur palladium sur charbon à 5 % et en opérant à 70°C sous 30 bars d'hydrogène pendant deux heures. On récupère 15,6 g d'un mélange solide d'amines de point de fusion 89°C. La phosgénation de 15 g de celui-ci, selon la description faite dans l'exemple 1 mène, après distillation du solvant, à 16,9 g d'un mélange d'isocyanates, teneur en NCO: 6,1 fonctions par kilo, qui se présente sous forme d'un produit noir pêteux pouvant être coulé à 55° - 58°C et qucorrespond aux formules générales I, II, III, IV de la description avec n = 2.

**Exemple 7**

La condensation de FRIEDEL et CRAFTS décrite dans l'exemple 5 conduit à un mélange contenant environ 85% en poids de produits à deux noyaux aromatiques et 15 % de produits plus lourds. On dissout 22,5 g de ce mélange brut dans 40 ml de chlorure de méthylène. En opérant selon la description faite dans l'exemple 1, on utilise un produit nitrant constitué de 15,5 g d'acide nitrique à 98,7 % et 20 ml d'acide sulfurique à 96 % et on récupère, après extraction, neutralisation et distillation du solvant, 28,4 g de mélange brut de nitration dont on hydrogène 20 g placés dans 0,2 l de méthanol en présence de 0,8 g de catalyseur palladium à 5 % sur charbon en opérant à 70°C sous une pression d'hydrogène de 35 bars pendant 1 h 45. Après filtration du catalyseur et distillation du méthanol, on recueille ainsi 16 g de produit brut brun foncé, d'aspect pâteux. La phosgénation de 15 g de celui-ci dans les conditions décrites dans l'exemple 1, donne, après distillation du solvant orthodichlorobenzène 17,3 g d'un liquide brun-foncé dont la teneur en fonctions isocyanates est de 6,7 équivalents par kilo et qui correspond aux formules générales I, II, III, IV avec n = 1 et 2.

**Revendications**

1 - Isocyanates aromatiques constitués par les mélanges d'isomères et correspondant à des structures hydrocarbonées dissymétriques diphénylméthane substituées, éventuellement associés à des structures dissymétriques diphényléthanes substituées, conformément aux formules générales:

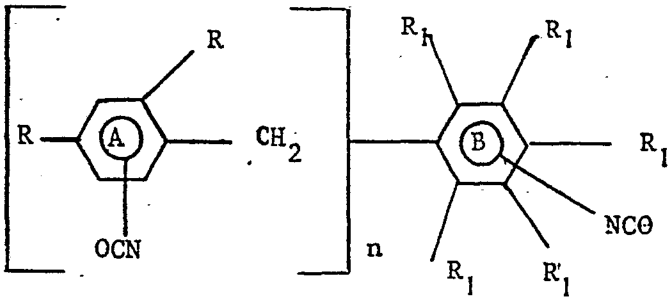 et 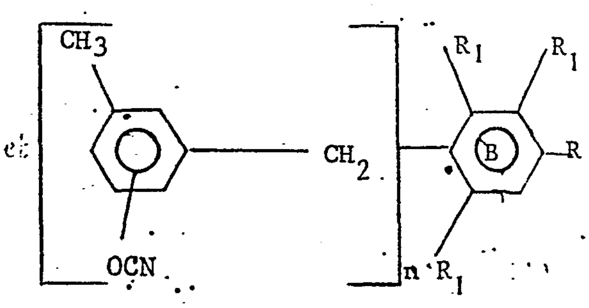

seules ou en mélange, éventuellement en association avec

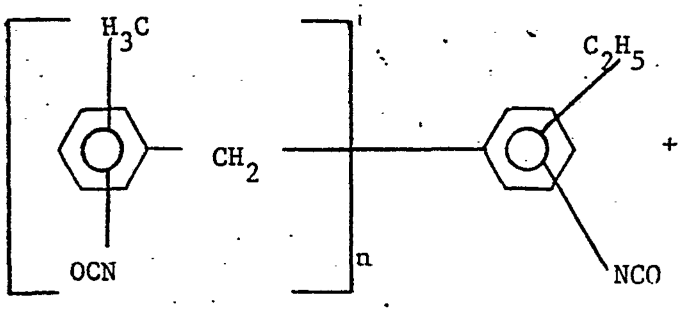 + 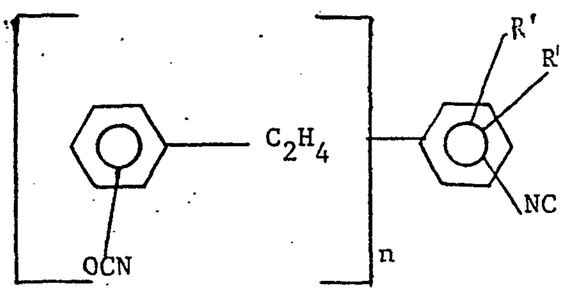

avec n de 1 a 2; R = H ou $CH_3$ et $R_1$ - H, $CH_3$ ou NCO; dans les noyaux de type A, un seul R correspondant à $CH_3$ et dans les noyaux de type B, un seul $R_1$ correspondant à NCO et deux $R_1$ correspondant à $CH_3$ sachant que sur ce noyau est fixee une fonction -NCO ne pouvant être entourée par des groupes méthyle sur les deux positions en ortho. R' = $CH_3$ ou $C_2H_5$; R'' = H ou $CH_3$ avec R'' = H si R' = $C_2H_5$ et R'' = $CH_3$ si R' = $CH_3$.

2. Isocyanates selon la revendication 1 caractérisés en ce qu'il s'agit des isomères de (parsméthyl benzyle isocyanate) paraxylène isoyanate.

3 - Isocyanates selon la revendication 1 caractérisés en ce qu'il s'agit des isomères d'(orthométhyl benzyle isocyanate) orthoxylène isocyanate.

4 - Isocyanates selon la revendication 1 caractérisés en ce qu'il s'agit das isomères de (métaméthyl benzyle isocyanate)méta xylène isocyanate.

5 - Isocyanates selon la revendication 1 caractérisés en ce qu'il s'agit des isomères d'(orthométhyl benzyle isocyanate)paraxylène isocyanate.

6 - Procédé de fabrication d'isocyanates aromatiques selon la revendication 1 caractérisé en ce que les composés hydrocarbonés de formule:

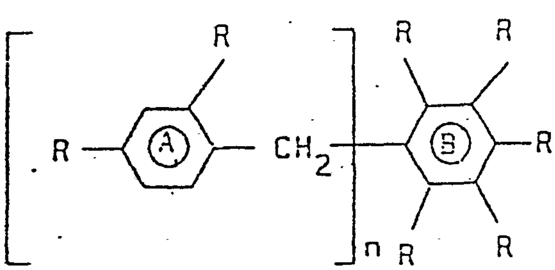 et 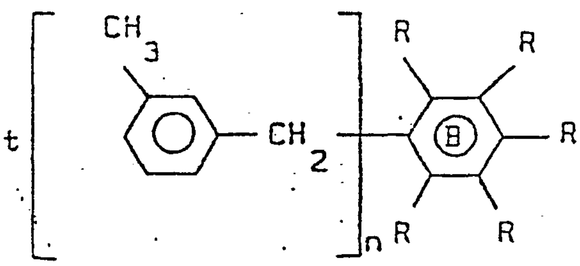

seules ou en mélange, éventuellement en association avec

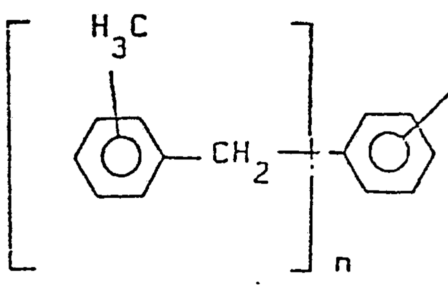
+
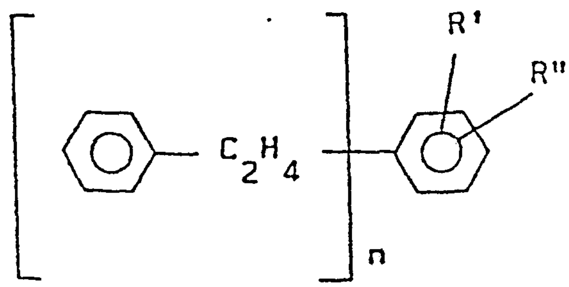

avec n de 1 à 2, R = H ou $CH_3$ tels que dans les noyaux de type A, un seul R correspond à $CH_3$ et dans les noyaux de type B, deux R correspondent à $CH_3$, R' = $CH_3$ ou $C_2H_5$ et R' = H ou $CH_3$ tels que R" = H si R' = $C_2H_5$ et R" = $CH_3$ si R' = $CH_3$, sont successivement nitrés, hydrogénés puis phosgénés.

7 - Procédé selon la revendication 6, caractérisé en ce que les composés hydrocarbonés à structure aromatique sont obtenus par condensation d'halogénures aromatiques substitués, préparés par halogénation photochimique du xylène, sur un excès de xylène.

8 - Procédé selon les revendications 6 ou 7 caractérisé en ce que la nitration s'effectue au moyen d'un mélange d'acide nitrique et d'acide sulfurique de concentrations supérieures à 85 %.

9 - Procédé selon l'une quelconque des revendications 6 à 8 caractérisé en ce que l'hydrogénation s'effectue en présence de palladium comme catalyseur.

**Patentansprüche**

1. Aromatische Isocyanate bestehend aus den Isomergemischen und entsprechend asymmetrischen Kohlenwasserstoff-Strukturen von substituierten Diphenylmethanen, gegebenenfalls kombiniert mit asymmetrischen Strukturen von substituierten Diphenylethanen, der allgemeinen Formeln

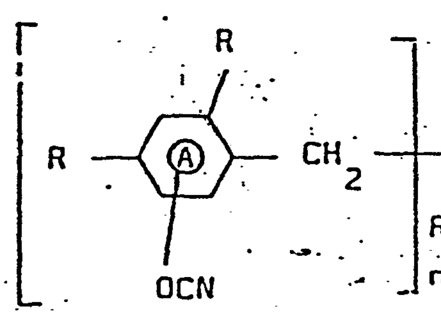
und
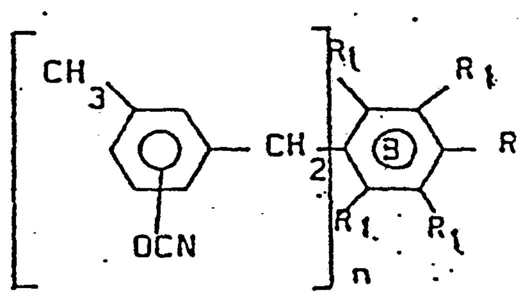

einzeln oder im Gemisch, gegebenenfalls kombiniert mit

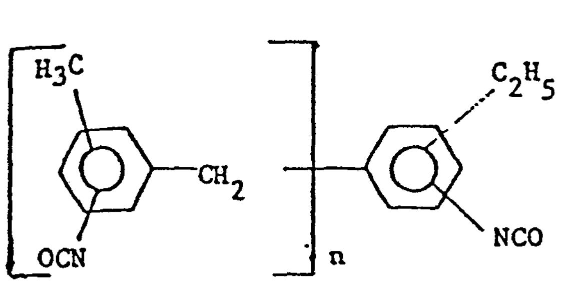
+
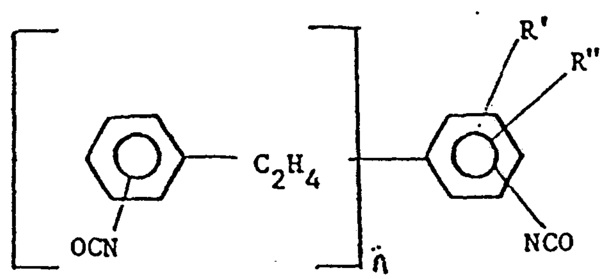

in denen n 1 bis 2 bedeutet; R = H oder $CH_3$ und $R_1$ = H, $CH_3$ oder NCO; und wobei in den Kernen vom Typ A nur ein R gleich $CH_3$ und in den Kernen vom Typ B nur ein $R_1$ gleich NCO ist und zwei $R_1$ die Gruppe $CH_3$ bedeuten, in Kenntnis, daß an àiesen Kern eine Cruppe -NCO gebunden ist, die nicht von Methylgruppen in den beiden 0-Stellungen umgeben sein kann, R' = $CH_3$ oder $C_2H_5$; R" = H oder $CH_3$ mit R' = H wenn R' =

$C_2H_5$ und R" = $CH_3$ wenn R' = $CH_3$.

2. Isocyanate nach Anspruch 8, dadurch gekennzeichnet, daß es sich um Isomere von (p-Methylbenzylisocyanat)-p-xylolisocyanat handelt.

3. Isocyanate nach Anspruch 1, dadurch gekennzeichnet, daß es sich um Isomere von (o-Methylbenzylisocyanat)-0-xylol-isocyanat handelt.

4. Isocyanate nach Anspruch 1, dadurch gekennzeichnet, daß es sich um Isomere von (m-Methylbenzylisocyanat)-m-xylolisocyanat handelt.

5. Isocyanate nach Anspruch 1, dadurch gekennzeichnet, daß daß es sich um Isomere von (o-Methylbenzylisocyanat)-p-xyoliisocyanat handelt.

6. Verfahren zur Herstellung von aromatischen Isocyanaten nach Anspruch I, dadurch gekennzeichnet, daß die Kohlenwasserstoffverbindungen der Formeln

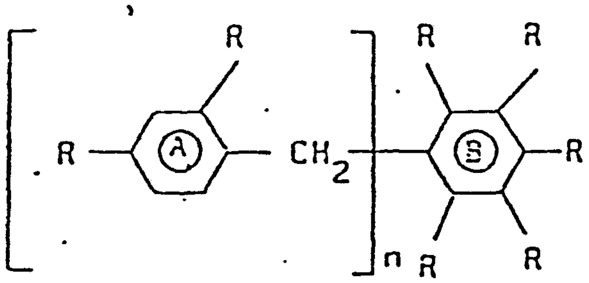

und

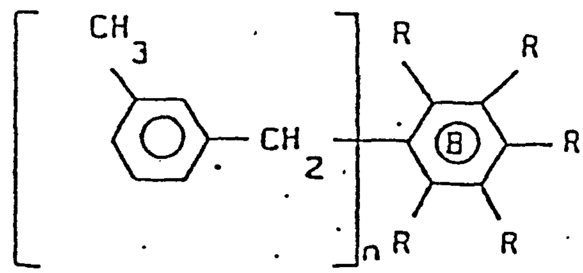

einzeln oder im Gemisch, gegebenenfalls in Kombination mit

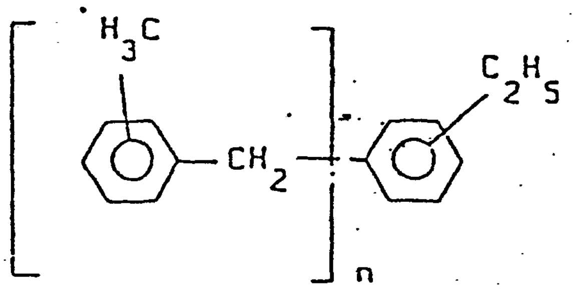

+

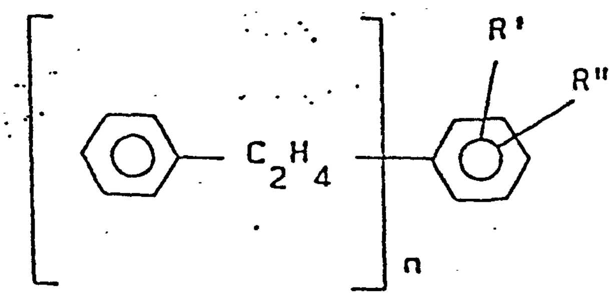

mit n = 1 bis 2, R = H oder $CH_3$ derart, daß in den Kernen vom Typ A ein einziges R gleich $CH_3$ ist und in den Kernen vom Typ B zwei R gleich $CH_3$ sind, R' = $CH_3$ oder $C_2H_5$ und R" = H oder $CH_3$ derart, daß R" = H wenn R' = $CH_3H_5$ und R" = $CH_3$ wenn R' = $CH_3$, nacheinander nitriert, hydriert und dann mit Phosgen umgesetzt werden.

7. Verfahren nach Anspruch 6, dadurch gekennzeichnet, daß die Kohlenwasserstoffverbindungen mit aromatischer Struktur durch Kondensation von substituierten aromatischen Halogeniden erhalten werden, die durch photochemische Halogenierung von Xylol mit einem Überschuß an Xylol erhalten worden sind.

8. Verfahren nach den Ansprüchen 6 oder 7, dadurch gekennzeichnet, daß die Nitrierung mit einem Gemisch aus Salpetersäure und Schwefelsäure in Konzentrationen oberhalb 85 % durchgeführt wird.

9. Verfahren nach einem der Ansprüche 6 bis 8, dadurch gekennzeichnet, daß die Hydrierung in Gegenwart von Palladium als Katalysator erfolgt.

**Claims**

1. Aromatic isocyanates consisting of mixtures of isomers and corresponding to substituted diphenylmethane asymmetric hydrocarbon structures, optionally in combination with substituted diphenylethane asymmetric structures, according to the general formulae:

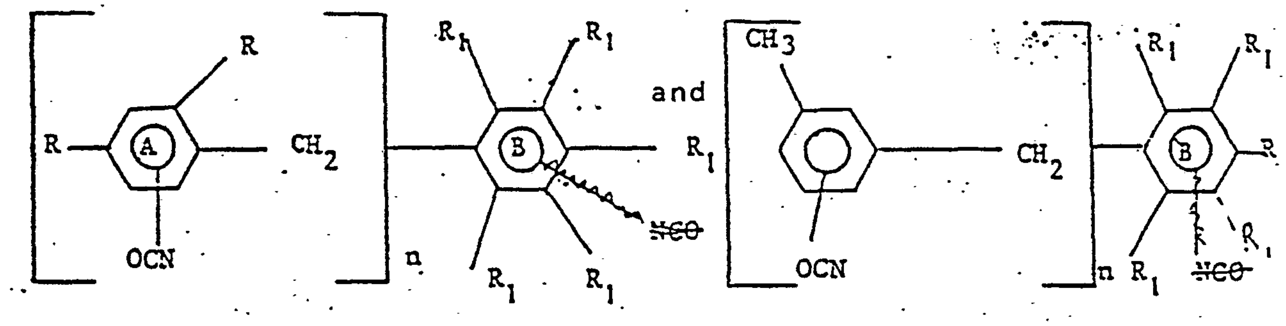

and

alone or mixed, optionally in combination with

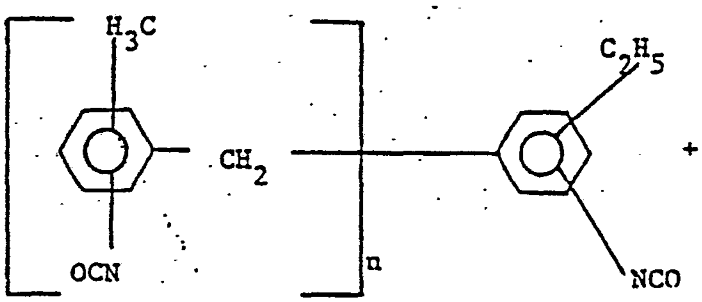

+

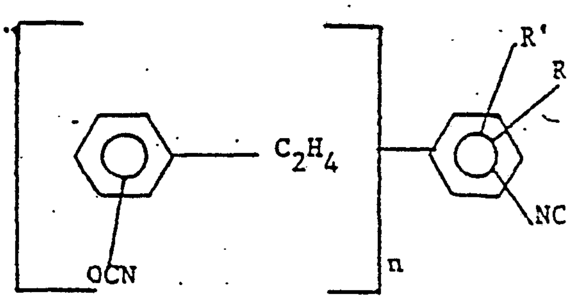

with n from 1 to 2; R = H or $CH_3$ and $R_1$ = H, $CH_3$ or NCO; in the rings of type A, a single R corresponding to $CH_3$ and in the rings of type B, a single $R_1$ corresponding to NCO and two groups Rl corresponding to $CH_3$, with the proviso that an -NCO group which cannot be surrounded by methyl groups in both ortho positions is bound to this ring. R' = $CH_3$ or $C_2H_5$; R" = H or $CH_3$, with $R_2$ = H if R' = $C_2H_5$ and R" = $CH_3$ if R' = $CH_3$.

2. Isocyanates according to Claim 1, characterized in that these are isomers of (para-methylbenzyl isocyanate)para-xylene isocyanate.

3. Isocyanates according to Claim 1, characterized in that these are isomers of (ortho-methylbenzyl isocyanate)-ortho-xylene isocyanate.

4. Isocyanates according to Claim 1, characterized in that these are isomers of (meta-methylbenzyl isocyanate)meta-xylene isocyanate.

5. Isocyanates according to Claim 1, characterized in that these are isomers of (ortho-methylbenzyl isocyanate)-para-xylene isocyanate.

6. Process for manufacturing aromatic isocyanates according to Claim 1, characterized in that the hydrocarbon compounds of formula:

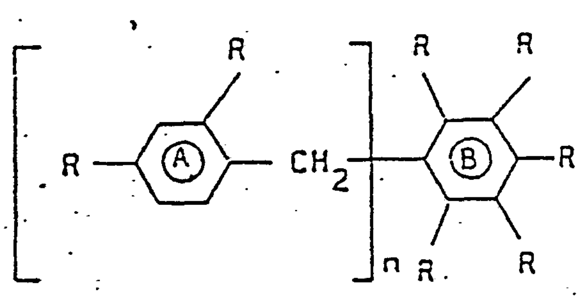

and

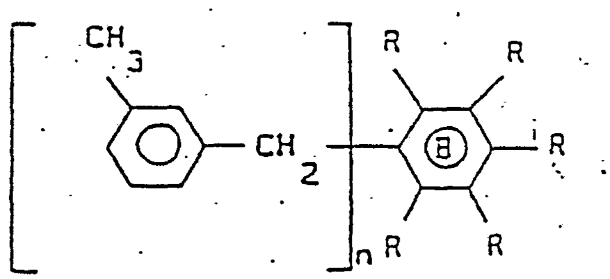

alone or mixed, and optionally in combination with

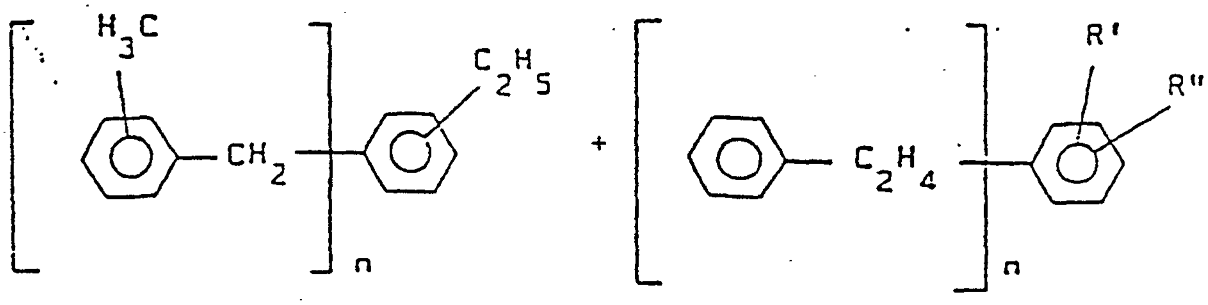

with n from 1 to 2, and R = H or $CH_3$ such that, in the rings of type A, a single R corresponds to $CH_3$ and, in the rings of type B, two groups R correspond to $CH_3$, R' = $CH_3$ or $C_2H_5$ and R" = H or $CH_3$ such that R" = H if R' = $C_2H_5$ and R" = $CH_3$ if R' = $CH_3$ are successively nitrated, hydrogenated and then treated with phosgene.

7. Process according to Claim 6, characterized in that the hydrocarbon compounds having an aromatic structure are obtained by condensation of substituted aromatic halides, prepared by photochemical halogenation of xylene, with an excess of xylene.

8. Process according to Claim 6 or 7, characterized in that the nitration is accomplished by means of a mixture of nitric acid and sulphuric acid of concentrations higher than 85%.

9. Process according to any one of Claims 6 to 8, characterized in that the hydrogenation is performed in the presence of palladium as a catalyst.